# EUROPÄISCHE PATENTSCHRIFT

(11) **EP 3 039 411 B1**
(45) Veröffentlichungstag und Bekanntmachung des Hinweises auf die Patenterteilung: **30.03.2022**
(21) Anmeldenummer: 14747798.8
(22) Anmeldetag: 30.06.2014
(51) Int. Cl.: G01N 21/87, G01N 21/55, G01N 21/47

(54) **ANORDNUNG ZUR ANALYSE EINES DURCH BRECHUNG UND REFLEXION AN EINEM SCHMUCKSTEIN HERVORGERUFENEN LICHTMUSTERS**
DEVICE FOR THE ANALYSIS OF A LIGHT PATTERN GENERATED BY REFRACTION AND REFLECTION AT A GEMSTONE
DISPOSITIF POUR L'ANALYSE D'UN MOTIF LUMINEUX GÉNÉRÉ PAR RÉFRACTION ET RÉFLEXION CHEZ UNE PIERRE PRÉCIEUSE

(30) Priorität: 27.08.2013 AT 6592013
(43) Veröffentlichungstag der Anmeldung: 06.07.2016
(73) Patentinhaber: D. Swarovski KG, 6112 Wattens (AT)
(72) Erfinder: EDER, Karlheinz, A-6220 Buch in Tirol (AT)
(74) Vertreter: Moore, Michael Richard
(86) Internationale Anmeldenummer: PCT/AT2014/000134
(87) Internationale Veröffentlichungsnummer: WO 2015/027252

(56) Entgegenhaltungen:
- EP-A1- 2 554 978
- EP-A2- 0 464 824
- US-A- 5 828 405

## Beschreibung

Die Erfindung betrifft eine Anordnung zur Analyse eines durch Brechung und Reflexion an einem Schmuckstein hervorgerufenen Lichtmusters, umfassend eine Lichtquelle zur Beleuchtung des Schmucksteins, eine Halterungsvorrichtung zur Halterung des Schmucksteins, einen, insbesondere flachen, Streuschirm zur Abbildung des Lichtmusters und eine Kamera zur Aufnahme des auf dem Streuschirm abgebildeten Lichtmusters. Die Erfindung betrifft darüber hinaus ein Verfahren zur Analyse eines durch Brechung und Reflexion an einem Schmuckstein hervorgerufenen Lichtmusters mittels der erfindungsgemäßen Anordnung.

EP2554978 veröffentlicht ein Verfahren zur Positionierung und Erkennung eines unsichtbaren Zeichens und Detektor dafür. Eine Anordnung mit den genannten Bestandteilen ist zum Beispiel aus der US 5,828,405 bekannt. Nachteilig bei dieser Anordnung gemäß dem Stand der Technik ist, dass die Beleuchtung des zu untersuchenden Diamanten über eine Öffnung im Streuschirm erfolgt. Dies hat zur Folge, dass der Teil des Lichtmusters, der im Bereich dieser Öffnung auf dem Streuschirm abgebildet werden würde, verloren geht. Darüber hinaus kann mit der in der US 5,828,405 offenbarten Beleuchtungsanordnung auch nur ein kleiner Teil des Diamanten beleuchtet werden. Auch auf diese Weise kommt es zu einem Verlust von Informationen über den Diamanten.

Und schließlich kommt als weiterer Nachteil hinzu, dass es aufgrund der Geometrie der Beleuchtungsanordnung erforderlich ist, die Kamera zur Aufnahme des auf dem Streuschirm abgebildeten Lichtmusters einerseits unter einem Winkel zum Schirm anzuordnen und andererseits relativ nahe zum Streuschirm zu positionieren. Letzteres erfordert den Einsatz eines extremen Fischaugenobjektivs. Insgesamt hat die Anordnung der Kamera in Bezug auf den Streuschirm bei der US 5,828,405 starke perspektivische Verzerrungen des aufgenommenen Lichtmusters zur Folge. Theoretisch könnte man zwar die Entfernung der Kamera zum Streuschirm erhöhen, sodass die Kamera zum Beispiel auf der Höhe des Edelsteins oder sogar dahinter angeordnet ist, allerdings wäre dann die freie Sicht auf den Streuschirm durch Teile der Beleuchtungsanordnung bzw. den Edelstein selber eingeschränkt, was wiederum zu einem Informationsverlust führen würde.

Beleuchtungsanordnung bzw. den Edelstein selber eingeschränkt, was wiederum zu einem Informationsverlust führen würde.

Der Vollständigkeit halber sei jedoch auch darauf hingewiesen, dass die Zielsetzung bei der US 5,828,405 darin besteht, einen charakteristischen "Fingerabdruck" eines Edelsteins aufzunehmen und mit einem in einer Datenbank hinterlegten Fingerabdruck zu vergleichen, um den Edelstein im Falle eines Diebstahl identifizieren zu können. Solange man das Bild des Edelsteins immer mit der gleichen Vorrichtung aufnimmt, spielen Informationsverluste und perspektivische Verzerrungen keine Rolle.

Im Gegensatz dazu kommt die Anordnung der gegenständlichen Erfindung zur Analyse eines durch Brechung und Reflexion an einem Schmuckstein hervorgerufenen Lichtmusters zum Einsatz. Hier geht es zum Beispiel darum, die Qualität eines Schmucksteins, die zum Beispiel durch Parameter wie die Geometrie oder die Politur beeinflusst wird, zu analysieren. Vor dem Hintergrund dieser Zielsetzung spielen die genannten Nachteile sehr wohl eine Rolle.

Es sei darauf hingewiesen, dass sich die gegenständliche Anordnung bzw. das gegenständliche Verfahren grundsätzlich zur Analyse aller Arten und Formen von Schmucksteinen verwenden lässt. Insbesondere können auch natürliche oder synthetisch hergestellte Schmuck- und Edelsteine, wie zum Beispiel Zirkonia oder aus Glas hergestellte Schmucksteine (Glassteine), analysiert werden.

Die Aufgabe der vorliegenden Erfindung besteht darin, die aus dem Stand der Technik bekannten Nachteile zu vermeiden und eine verbesserte Anordnung zur Analyse eines durch Brechung und Reflexion an einem Schmuckstein hervorgerufenen Lichtmusters bzw. ein verbessertes Verfahren hierzu, bei dem die erfindungsgemäße Anordnung zum Einsatz kommt, bereitzustellen.

Diese Aufgabe wird durch die Merkmale der unabhängigen Ansprüche 1 und 11 gelöst.

Einer der Kerngedanken der vorliegenden Erfindung besteht also darin, dass die Anordnung ein halbdurchlässiges optisches Element zur Umlenkung des von der Lichtquelle ausgehenden Lichts in Richtung des Schmucksteins und Transmission des an dem Schmuckstein gebrochenen und reflektierten Lichts umfasst, wobei es sich bei dem halbdurchlässigen optischen Element zum Beispiel um einen halbdurchlässigen Spiegel oder eine Glasplatte handeln kann.

Einer der wesentlichen Unterschiede gegenüber dem Stand der Technik besteht also darin, dass die Lichteinkoppelung über ein halbdurchlässiges optisches Element erfolgt. Auf diese Weise ist es möglich, die Elemente der Anordnung flexibler anzuordnen und auf diese Weise die aus dem Stand der Technik bekannten Nachteile zu vermeiden:
Beispielsweise können die Halterungsvorrichtung zur Halterung des Schmucksteins und die Lichtquelle zur Beleuchtung des Schmucksteins auf der gleichen Seite des Streuschirms angeordnet werden. Dies hat den Vorteil, dass zur Beleuchtung des Schmucksteins kein Loch im Streuschirm vorgesehen sein muss. Gleichzeitig wird auch Platz geschaffen für eine im Wesentlichen senkrechte Anordnung der Kamera zur Aufnahme des auf dem Streuschirm abgebildeten Lichtmusters relativ zum Streuschirm. Hierzu kann es vorgesehen sein, dass die Kamera auf der der Halterungsvorrichtung gegenüberliegenden Seite des Streuschirms angeordnet ist und/oder es sich bei dem Streuschirm um einen Transmissionsschirm handelt. Durch eine derartige Anordnung der Kamera in Bezug auf den Streuschirm ist es möglich, den Abstand der Kamera zum Streuschirm so zu wählen, dass Objektive eingesetzt werden können, die keine oder nur sehr geringe perspektivische Verzerrungen des abgebildeten Lichtmusters zur Folge haben. Außerdem können Informationsverluste gänzlich vermieden werden, da die Notwendigkeit entfällt, zwischen der Kamera und dem Streuschirm Elemente anzuordnen, die die freie Sicht der Kamera auf den Streuschirm beeinträchtigen.

Der scheinbare Nachteil durch die Verwendung eines zusätzlichen optischen Elements in Form eines halbdurchlässigen optischen Elements (jedes optische Element ist immer mit mehr oder weniger stark ausgeprägten Abbildungsfehlern verbunden) wird also durch eine Reihe von Vorteilen mehr als ausgeglichen.

In einer vorteilhaften Ausbildung der erfindungsgemäßen Anordnung ist die Halterungsvorrichtung in einem Abstand zwischen 10 cm und 50 cm, vorzugsweise in einem Abstand von in etwa 30 cm vom Streuschirm angeordnet.

Weiterhin hat es sich als vorteilhaft herausgestellt, dass die Kamera einen CCD-Chip umfasst, und/oder dass es sich bei der Lichtquelle um eine LED-Lichtquelle handelt.

Das von der Lichtquelle ausgehende Licht ist vorzugsweise unpolarisiert, und/oder weist einen Strahldurchmesser zwischen 5 mm und 15 mm, vorzugsweise einen Strahldurchmesser von in etwa 10 mm, auf, und/oder ist kollimiert, und/oder weist einen Divergenzhalbwinkel zwischen 0,1° und 0,4°, vorzugsweise einen Divergenzhalbwinkel von in etwa 0,25°, auf, und/oder weist eine Spektrum mit einer Vielzahl unterschiedlicher Wellenlängen aus dem Bereich des sichtbaren Lichts auf. Letzeres äußert sich darin, dass der Schmuckstein im Wesentlichen mit weißem Licht beleuchtet wird, das durch die Brechung an dem Schmuckstein zum Teil in seine Spektralfarben zerlegt wird, sodass das auf dem Streuschirm abgebildete Lichtmuster zum Teil farbige Lichtreflexe umfasst.

Bevorzugt ist es auch vorgesehen, dass die erfindungsgemäße Anordnung einer Auswerteeinheit, vorzugsweise einen Computer, zur Auswertung des von der Kamera aufgenommenen Lichtmusters umfasst, und/oder dass zumindest die Lichtquelle, die Halterungsvorrichtung, der Streuschirm, die Kamera und das halbdurchlässige optische Element in einer Vorrichtung zur Unterdrückung von Hintergrundlicht angeordnet sind. Durch letztere Maßnahme kann das Signal-Rausch-Verhältnis deutlich verbessert werden.

Wie eingangs ausgeführt, wird Schutz auch begehrt für ein Verfahren zur Analyse eines durch Brechung und Reflexion an einem Schmuckstein hervorgerufenen Lichtmusters mittels einer erfindungsgemäßen Anordnung, wobei das Verfahren die folgenden Verfahrensschritte umfasst: Halterung des zu analysierenden Schmucksteins in der Halterungsvorrichtung, Beleuchtung des Schmucksteins mittels der Lichtquelle und Aufnahme des auf dem Streuschirm abgebildeten Lichtmusters mittels der Kamera.

In einer besonders bevorzugten Ausführungsform dieses Verfahrens ist es vorgesehen, dass die Verfahrensschritte 2 und 3 wenigstens einmal mit einer unterschiedlichen Belichtungszeit und/oder mit einer unterschiedlichen Intensität und/oder mit einer unterschiedlichen Polarisation (im Falle, dass polarisiertes Licht zum Einsatz kommt) wiederholt werden. In weiterer Folge ist es dann nämlich möglich - falls als weiterer Verfahrensschritt die Auswertung des von der Kamera aufgenommenen Lichtmusters mittels der Auswerteeinheit umfasst ist - aus wenigstens zwei bei unterschiedlichen Belichtungszeiten und/oder Intensitäten und/oder Polarisationen aufgenommenen Lichtmustern ein HDR-Bild mit einem erhöhten Informationsgehalt zu erzeugen.

Und schließlich hat es sich als günstig herausgestellt, das von der Kamera aufgenommene Lichtmuster mit wenigstens einem in der Auswerteeinheit hinterlegten Lichtmuster zu vergleichen, wobei es sich bei dem hinterlegten Lichtmuster vorzugsweise um ein simuliertes Lichtmuster handelt. Auf diese Weise können zum Beispiel Abweichungen von einem idealen Schliff des Schmucksteins detektiert werden.

Weitere Merkmale und Vorteile der vorliegenden Erfindung ergeben sich aus den Figuren und der folgenden Figurenbeschreibung. Dabei zeigt
- Fig. 1: eine bevorzugte Ausführungsform der Anordnung in einer schematischen Darstellung,
- Fig. 2: exemplarisch ein durch Brechung und Reflexion an einem Schmuckstein hervorgerufenes und auf dem Streuschirm abgebildetes Lichtmuster in einer vereinfachten schematischen Darstellung und
- Fig. 3: ein Flussdiagramm zur schematischen Darstellung einer bevorzugten Ausführungsform des erfindungsgemäßen Verfahrens.

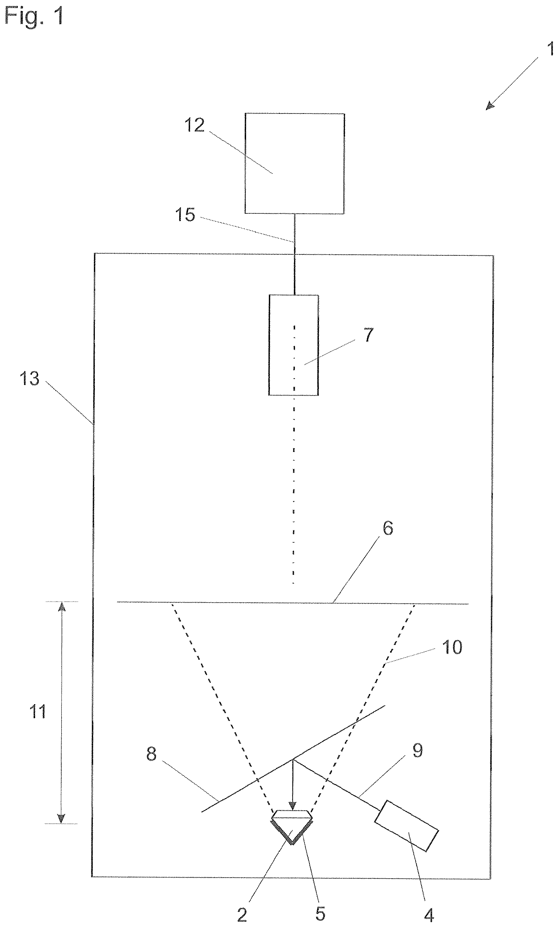

Die in Figur 1 gezeigte bevorzugte Ausführungsform der erfindungsgemäßen Anordnung 1 zur Analyse eines durch Brechung und Reflexion an einem Schmuckstein hervorgerufenen Lichtmusters umfasst eine Lichtquelle 4 zur Beleuchtung eines in einer Halterungsvorrichtung 5 gehalterten Schmucksteins 2. Als Lichtquelle 4 kommt dabei eine LED-Lichtquelle zum Einsatz. Das von der Lichtquelle 4 ausgehende Licht - die entsprechende optische Achse ist mit dem Bezugszeichen 9 versehen - ist unpolarisiert, weist einen Strahldurchmesser von in etwa 10 mm auf, ist kollimiert und weist einen Divergenzhalbwinkel von in etwa 0,25° sowie ein Spektrum mit einer Vielzahl unterschiedlicher Wellenlängen aus dem Bereich des sichtbaren Lichts auf. Aufgrund der Größe des Strahldurchmessers können die meisten handelsüblichen Schmucksteine komplett ausgeleuchtet werden.

Das von der Lichtquelle 4 ausgehende Licht 9 trifft zunächst unter einem Winkel auf ein halbdurchlässiges optisches Element 8 in Form eines halbdurchlässigen Spiegels und wird an der der Lichtquelle zugewandten Oberfläche des halbdurchlässigen optischen Elements 8 reflektiert. Genauer gesagt findet eine Umlenkung des Lichts in Richtung des Schmucksteines 2 statt. Ein Teil des auf den Schmuckstein 2 auftreffenden Lichts wird dabei sofort in Richtung Streuschirm 6 reflektiert. Der übrige - weitaus größere-Teil des Lichts tritt in den Schmuckstein 2 ein, wird dort gebrochen und erfährt Totalreflexionen an den Flächen des facettierten Schmucksteins 2, bis es schließlich wieder aus dem Schmuckstein 2 in Richtung Streuschirm 6 austritt. Ein Teil des Lichts verlässt den Schmuckstein 2 natürlich auch in eine dem Streuschirm 6 abgewandte Richtung und geht damit im Hinblick auf das Lichtmuster "verloren".

Das von dem zu analysierenden Schmuckstein 2 ausgehende gebrochene und reflektierte Licht 10 - angedeutet durch die gestrichelten Linien - passiert in Transmission das halbdurchlässige optisch Element 8 und trifft dann auf den Streuschirm 6, auf dem ein je nach Art des Schmucksteins 2 charakteristisches Streubild abgebildet wird. Der Abstand der Halterungsvorrichtung 5 und damit (in etwa der Mitte) des Schmucksteins 2 zum Streuschirm 6 beträgt ca. 30 cm.

Wie anhand des in der Figur 1 dargestellten Aufbaus der Anordnung 1 erkennbar ist, sind die Halterungsvorrichtung 5 und die Lichtquelle 4 auf der gleichen Seite des Streuschirms 6 angeordnet.

Bei dem Streuschirm 6 handelt es sich um einen Transmissionsschirm, das heißt, dass das auf dem Streuschirm 6 abgebildete Lichtmuster auch auf der der Halterungsvorrichtung 5 abgewandten Seite des Streuschirms 6 sichtbar ist. Auf diese Weise ist es möglich, das auf dem Streuschirm 6 abgebildete Lichtmuster mittels einer Kamera 7 aufzunehmen, die auf der der Halterungsvorrichtung 5 gegenüberliegenden Seite des Streuschirms 6 angeordnet ist. Darüber hinaus ist es auch möglich, die Kamera 7 in ihrer Längserstreckung im Wesentlichen senkrecht zum Streuschirm 6 auszurichten, was durch die strichpunktierte Linie angedeutet ist. Bei der Kamera 7 handelt es sich um eine CCD-Kamera mit einem entsprechenden CCD-Chip.

Die Lichtquelle 4, die Halterungsvorrichtung 5, der Streuschirm 6, die Kamera 7 und das halbdurchlässige optische Element 8 sind in einer Vorrichtung 13 zur Unterdrückung von Hintergrundlicht angeordnet, wobei die Wandungen dieser Vorrichtung 13 eine schwarze, matte Oberfläche aufweisen.

Die Kamera 7 ist über eine geeignete Datenleitung 15, die auch drahtlos ausgebildet sein kann, mit einer Auswerteeinheit 12 in Form eines Computers verbunden.

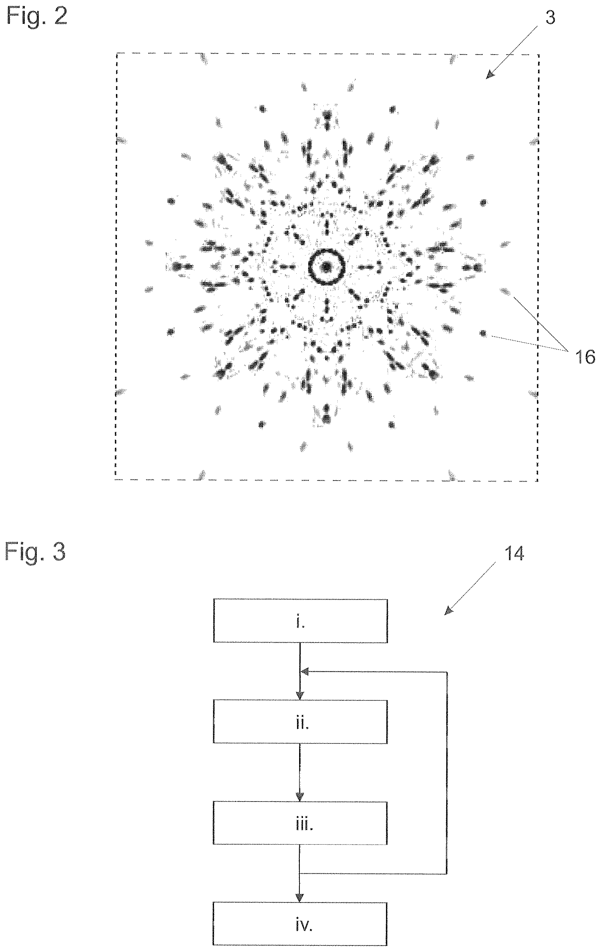

Figur 2 zeigt in einer Schwarz-Weiß-Darstellung exemplarisch ein durch Brechung und Reflexion an einem Schmuckstein hervorgerufenes und auf dem Streuschirm abgebildetes Lichtmuster 3. Dieses Lichtmuster 3 setzt sich aus einzelnen Lichtflecken 16 zusammen, wobei diese Lichtflecke 16 für jeden Schmuckstein charakteristisch durch Brechung und Reflexion erzeugt werden. Es sei darauf hingewiesen, dass es sich bei dem in der Figur 2 gezeigten Bild um ein Falschfarbenbild mit invertierter Helligkeit handelt. Die weißen Bereiche sind in Wirklichkeit schwarz, das heißt hier gelangt kein Licht hin, und die dunklen Bereiche sind diejenigen Bereiche des Streuschirms, auf die Licht trifft. Durch die vereinfachte Schwarz-Weiß-Darstellung ist ebenfalls nicht erkennbar, dass die Lichtflecke 16 zum Teil einen Farbverlauf aufweisen, der der Zerlegung von weißem Licht durch ein Prisma ähnelt. Es sei auch noch darauf hingewiesen, dass die Lichtflecke 16 eine unterschiedliche Helligkeit aufweisen können. In der englischsprachigen Literatur wird ein solches Lichtmuster eines Schmucksteins häufig auch als "Fire" bezeichnet.

In der Figur 3 ist anhand eines Flussdiagramms eine bevorzugte Ausführungsform des erfindungsgemäßen Verfahrens 14 dargestellt: In einem ersten Schritt i. wird ein zu analysierender Schmuckstein in der Halterungsvorrichtung gehaltert. In einem sich anschließenden Verfahrensschritt ii. erfolgt die Beleuchtung des Schmucksteins mittels der Lichtquelle. In einem dritten Verfahrensschritt iii. wird das auf dem Streuschirm abgebildete Lichtmuster mittels der Kamera aufgenommen. Die Verfahrensschritte ii. und iii. werden anschließend mit einer Reihe, zum Beispiel zehn, unterschiedlicher Belichtungszeiten, rangierend beispielsweise von ca. 60 ms bis ca. 16000 ms wiederholt, das heißt es werden nacheinander mehrere Lichtmuster mittels der Kamera aufgenommen, die bei unterschiedlichen Belichtungszeiten entstehen.

In einem weiteren Verfahrensschritt iv. erfolgt die Auswertung der von der Kamera aufgenommenen Lichtmuster mittels der Auswerteeinheit. Hiezu wird zunächst aus den bei unterschiedlichen Belichtungszeiten aufgenommenen Lichtmustern ein HDR-Bild (High Dynamic Range) erzeugt und anschließend mit einem in der Auswerteeinheit hinterlegten Lichtmuster, bei dem es sich um ein simuliertes Lichtmuster handelt, verglichen. Anhand dieses Vergleichs können Rückschlüsse auf die Qualität des Schmucksteins gezogen werden.

## Patentansprüche

1. Anordnung (1) zur Analyse eines durch Brechung und Reflexion an einem Schmuckstein (2) hervorgerufenen Lichtmusters (3), umfassend
- eine Lichtquelle (4) zur Beleuchtung des Schmucksteins (2),
eine Halterungsvorrichtung (5) zur Halterung des Schmucksteins (2),
einen, Streuschirm (6) zur Abbildung des Lichtmusters (3) und
eine Kamera (7) zur Aufnahme des auf dem Streuschirm (6) abgebildeten Lichtmusters (3),
**dadurch gekennzeichnet, dass** die Anordnung (1) ein halbdurchlässiges optisches Element (8) zur Umlenkung des von der Lichtquelle (4) ausgehenden Lichts (9) in Richtung des Schmucksteins (2) und Transmission des an dem Schmuckstein (2) gebrochenen und reflektierten Lichts (10) umfasst.

2. Anordung (1) nach Anspruch 1, **dadurch gekennzeichnet, dass** der Streuschirm (6) flach ist.

3. Anordnung (1) nach Anspruch 1 oder 2, **dadurch gekennzeichnet, dass** es sich bei dem halbdurchlässigen optischen Element (8) um einen halbdurchlässigen Spiegel oder eine Glasplatte handelt.

4. Anordnung (1) nach einem der Ansprüche 1 bis 3, **dadurch gekennzeichnet, dass** die Halterungsvorrichtung (5) und die Lichtquelle (4) auf der gleichen Seite des Streuschirms (6) angeordnet sind.

5. Anordnung (1) nach einem der Ansprüche 1 bis 4, **dadurch gekennzeichnet, dass** die Kamera (7) auf der der Halterungsvorrichtung (5) gegenüberliegenden Seite des Streuschirms (6) angeordnet ist und/oder im Wesentlichen senkrecht zum Streuschirm (6) ausgerichtet ist und/oder es sich bei dem Streuschirm um einen Transmissionsschirm handelt.

6. Anordnung (1) nach einem der Anspruche 1 bis 5, **dadurch gekennzeichnet, dass** die Halterungsvorrichtung (5) in einem Abstand (11) zwischen 10cm und 50cm, vorzugsweise in einem Abstand (11) von in etwa 30cm, vom Streuschirm (6) angeordnet ist.

7. Anordnung (1) nach einem der Anspruche 1 bis 6, **dadurch gekennzeichnet, dass** es sich bei der Lichtquelle (4) um eine LED-Lichtquelle handelt.

8. Anordnung (1) nach einem der Ansprüche 1 bis 7, **dadurch gekennzeichnet, dass** das von der Lichtquelle (4) ausgehende Licht (9)
unpolarisiert ist, und/oder
einen Strahldurchmesser zwischen 5mm und 15mm, vorzugsweise einen Strahldurchmesser von in etwa 10mm, aufweist, und/oder kollimiert ist, und/oder
einen Divergenzhalbwinkel zwischen 0,1° und 0,4°, vorzugsweise einen Divergenzhalbwinkel von in etwa 0,25°, aufweist, und/oder ein Spektrum mit einer Vielzahl unterschiedlicher Wellenlangen aus dem Bereich des sichtbaren Lichts aufweist.

9. Anordnung (1) nach einem der Ansprüche 1 bis 8, **dadurch gekennzeichnet, dass** die Anordnung (1) eine Auswerteeinheit (12), vorzugsweise einen Computer, zur Auswertung des von der Kamera (7) aufgenommenen Lichtmusters (3) umfasst.

10. Anordnung (1) nach einem der Ansprüche 1 bis 9, **dadurch gekennzeichnet, dass** zumindest die Lichtquelle (4), die Halterungsvorrichtung (5), der Streuschirm (6), die Kamera (7) und das halbdurchlässige optische Element (8) in einer Vorrichtung (13) zur Unterdrückung von Hintergrundlicht angeordnet sind.

11. Verfahren (14) zur Analyse eines durch Brechung und Reflexion an einem Schmuckstein (2) hervorgerufenen Lichtmusters (3) mittels einer Anordnung (1) nach einem der Ansprüche bis 10, umfassend die folgenden Verfahrensschritte:
i. Halterung des zu analysierenden Schmucksteins (2) in der Halterungsvorrichtung (5),
ii. Beleuchtung des Schmucksteins (2) mittels der Lichtquelle (4) und
iii. Aufnahme des auf dem Streuschirm (6) abgebildeten Lichtmusters (3) mittels der Kamera (7).

12. Verfahren (14) nach Anspruch 11, wobei die Verfahrensschritte ii. und iii. wenigstens einmal mit einer unterschiedlichen Belichtungszeit und/oder mit einer unterschiedlichen Intensität und/oder mit einer unterschiedlichen Polarisation wiederholt werden.

13. Verfahren (14) nach Anspruch 11 oder 12, wobei als weiterer Verfahrensschritt (iv.) die Auswertung des von der Kamera (7) aufgenommenen Lichtmusters (3) mittels der Auswerteeinheit (12) umfasst ist.

14. Verfahren (14) nach Anspruch 13, wobei aus wenigstens zwei bei unterschiedlichen Belichtungszeiten und/oder Intensitäten und/oder Polarisationen aufgenommenen Lichtmustern ein HDR-Bild erzeugt wird.

15. Verfahren (14) nach Anspruch 13 oder 14, wobei das von der Kamera (7) aufgenommene Lichtmuster (3) mit wenigstens einem in der Auswerteeinheit (12) hinterlegten Lichtmuster verglichen wird, wobei es sich bei dem hinterlegten Lichtmuster vorzugsweise um ein simuliertes Lichtmusterhandelt.

## Claims

1. Assembly (1) for analysing a light pattern (3) caused by refraction and reflection at a gemstone (2), comprising
- a light source (4) for illuminating the gemstone (2),
a holding device (5) for holding the gemstone (2),
a diffusing screen (6) for imaging the light pattern (3), and
a camera (7) for capturing the light pattern (3) imaged on the diffusing screen (6),
**characterised in that** the assembly (1) comprises a semi-transparent optical element (8) for deflecting the light (9) emitted by the light source (4) in the direction of the gemstone (2) and for transmitting the light (10) which is refracted and reflected at the gemstone (2).

2. Assembly (1) according to claim 1, **characterised in that** the diffusing screen (6) is planar.

3. Assembly (1) according to either claim 1 or claim 2, **characterised in that** the semi-transparent optical element (8) is a semi-transparent mirror or a glass plate.

4. Assembly (1) according to any of claims 1 to 3, **characterised in that** the holding device (5) and the light source (4) are arranged on the same side of the diffusing screen (6).

5. Assembly (1) according to any of claims 1 to 4, **characterised in that** the camera (7) is arranged on the side of the diffusing screen (6) opposite the holding device (5) and/or is oriented substantially perpendicularly to the diffusing screen (6), and/or the diffusing screen is a transmission screen.

6. Assembly (1) according to any of claims 1 to 5, **characterised in that** the holding device (5) is arranged at a distance (11) of between 10 cm and 50 cm, preferably at a distance (11) of approximately 30 cm, from the diffusing screen (6).

7. Assembly (1) according to any of claims 1 to 6, **characterised in that** the light source (4) is an LED light source.

8. Assembly (1) according to any of claims 1 to 7, **characterised in that** the light (9) emitted by the light source (4)
is unpolarised, and/or
has a beam diameter of between 5 mm and 15 mm, preferably a beam diameter of approximately 10 mm, and/or is collimated, and/or
has a divergence half angle of between 0.1° and 0.4°, preferably a divergence half angle of approximately 0.25°, and/or
has a spectrum having a plurality of different wavelengths from the visible light range.

9. Assembly (1) according to any of claims 1 to 8, **characterised in that** the assembly (1) comprises an evaluation unit (12), preferably a computer, for evaluating the light pattern (3) captured by the camera (7).

10. Assembly (1) according to any of claims 1 to 9, **characterised in that** at least the light source (4), the holding device (5), the diffusing screen (6), the camera (7) and the semi-transparent optical element (8) are arranged in a device (13) for suppressing background light.

11. Method (14) for analysing a light pattern (3) caused by refraction and reflection at a gemstone (2) by means of an assembly (1) according to any of the claims up to 10, comprising the following method steps:
i. holding the gemstone (2) to be analysed in the holding device (5),
ii. illuminating of the gemstone (2) by means of the light source (4) and
iii. capturing the light pattern (3) imaged on the diffusing screen (6) by means of the camera (7).

12. Method (14) according to claim 11, wherein method steps ii. and iii. are repeated at least once using a different exposure time and/or a different intensity and/or a different polarisation.

13. Method (14) according to either claim 11 or claim 12, wherein evaluating the light pattern (3) captured by the camera (7) by means of the evaluation unit (12) is included as a further method step (iv.).

14. Method (14) according to claim 13, wherein an HDR image is generated from at least two light patterns captured using different exposure times and/or intensities and/or polarisations.

15. Method (14) according to either claim 13 or claim 14, wherein the light pattern (3) captured by the camera (7) is compared with at least one light pattern stored in the evaluation unit (12), wherein the stored light pattern is preferably a simulated light pattern.

## Revendications

1. Système (1) servant à analyser un motif lumineux (3) généré par réfraction et réflexion sur une pierre précieuse (2), comprenant
- une source lumineuse (4) servant à éclairer la pierre précieuse (2),
un dispositif de support (5) servant à supporter la pierre précieuse (2),
un écran de diffusion (6) servant à reproduire le motif lumineux (3) et
une caméra (7) servant à enregistrer le motif lumineux (3) reproduit sur l'écran de diffusion (6),
**caractérisé en ce que** le système (1) comprend un élément optique semi-transparent (8) servant à dévier la lumière (9) émise par la source lumineuse (4) en direction de la pierre précieuse (2) et à transmettre la lumière (10) réfractée et réfléchie à la pierre précieuse (2).

2. Système (1) selon la revendication 1, **caractérisé en ce que** l'écran de diffusion (6) est plat.

3. Système (1) selon la revendication 1 ou 2, **caractérisé en ce que** l'élément optique semi-transparent (8) est un miroir semi-transparent ou une plaque de verre.

4. Système (1) selon l'une des revendications 1 à 3, **caractérisé en ce que** le dispositif de support (5) et la source lumineuse (4) sont disposés du même côté de l'écran de diffusion (6).

5. Système (1) selon l'une des revendications 1 à 4, **caractérisé en ce que** la caméra (7) est disposée sur le côté de l'écran de diffusion (6) opposé au dispositif de support (5) et/ou est orientée sensiblement perpendiculairement à l'écran de diffusion (6) et/ou l'écran de diffusion est un écran de transmission.

6. Système (1) selon l'une des revendications 1 à 5, **caractérisé en ce que** le dispositif de support (5) est disposé à une distance (11) comprise entre 10 cm et 50 cm, de préférence à une distance (11) d'environ 30 cm, de l'écran de diffusion (6).

7. Système (1) selon l'une des revendications 1 à 6, **caractérisé en ce que** la source lumineuse (4) est une source lumineuse à DEL.

8. Système (1) selon l'une des revendications 1 à 7, **caractérisé en ce que** la lumière (9) émise par la source lumineuse (4)
est non polarisée, et/ou
présente un diamètre de faisceau compris entre 5 mm et 15 mm, de préférence un diamètre de faisceau d'environ 10 mm, et/ou est collimatée, et/ou
présente un demi-angle de divergence compris entre 0,1° et 0,4°, de préférence un demi-angle de divergence d'environ 0,25°, et/ou
présente un spectre comportant une pluralité de longueurs d'onde différentes du domaine de la lumière visible.

9. Système (1) selon l'une des revendications 1 à 8, **caractérisé en ce que** le système (1) comprend une unité d'évaluation (12), de préférence un ordinateur, servant à évaluer le motif lumineux (3) enregistré par la caméra (7).

10. Système (1) selon l'une des revendications 1 à 9, **caractérisé en ce qu'**au moins la source lumineuse (4), le dispositif de support (5), l'écran de diffusion (6), la caméra (7) et l'élément optique semi-transparent (8) sont disposés dans un dispositif (13) pour supprimer la lumière de fond.

11. Procédé (14) servant à analyser un motif lumineux (3) généré par réfraction et réflexion sur une pierre précieuse (2) au moyen d'un système (1) selon l'une des revendications jusqu'à 10, comprenant les étapes de procédé suivantes :
i. support de la pierre précieuse (2) à analyser dans le dispositif de support (5),
ii. éclairage de la pierre précieuse (2) au moyen de la source lumineuse (4) et
iii. enregistrement du motif lumineux (3) reproduit sur l'écran de diffusion (6) au moyen de la caméra (7).

12. Procédé (14) selon la revendication 11, dans lequel les étapes de procédé ii. et iii. sont répétées au moins une fois avec un temps d'exposition différent et/ou avec une intensité différente et/ou avec une polarisation différente.

13. Procédé (14) selon la revendication 11 ou 12, dans lequel l'évaluation du motif lumineux (3) enregistré par la caméra (7) au moyen de l'unité d'évaluation (12) est comprise en tant qu'étape de procédé supplémentaire (iv.).

14. Procédé (14) selon la revendication 13, dans lequel une image HDR est générée à partir d'au moins deux motifs lumineux enregistrés avec des temps d'exposition et/ou des intensités et/ou des polarisations différents.

15. Procédé (14) selon la revendication 13 ou 14, dans lequel le motif lumineux (3) enregistré par la caméra (7) est comparé à au moins un motif lumineux stocké dans l'unité d'évaluation (12), le motif lumineux stocké étant de préférence un motif lumineux simulé.
